# EUROPEAN PATENT APPLICATION

(11) **EP 4 792 744 A1**
(43) Date of publication of application: **19.08.2026**
(21) Application number: 25158260.7
(22) Date of filing: 17.02.2025
(51) Int. Cl.: A61B 6/00

(54) **DETERMINING PRESENCE OF CONTRAST AGENT FLOW ARTIFACTS**

(71) Applicant: Koninklijke Philips N.V., 5656 AE Eindhoven (NL)
(72) Inventor: DAERR, Heiner, Eindhoven (NL); KOEHLER, Thomas, Eindhoven (NL)
(74) Representative: Philips Intellectual Property & Standards

(57) **Abstract**

A system (100) for determining a presence of contrast agent flow artifacts, is provided. The system includes one or more processors (110) configured to receive (S110) X-ray attenuation data (120), the X-ray attenuation data representing, at each of a plurality of different points in time (i), a spatial distribution (130ᵢ) of contrast agent within a vascular region. The one or more processors (110) are also configured to analyze (S120) the X-ray attenuation data (120) to identify one or more differences (140ⱼ) between the spatial distributions (130ᵢ). The one or more processors (110) are also configured to determine (S130) a presence of at least one contrast agent flow artifact (150) in the vascular region based on the one or more differences (140ⱼ).

## Description

### TECHNICAL FIELD

The present disclosure relates to determining the presence of contrast agent flow artifacts. A system, a computer-implemented method, and a computer program product, are disclosed.

### BACKGROUND

Contrast agent is routinely used to enhance medical images that are acquired using X-ray radiation. The contrast agent is typically injected into the vasculature, e.g. in the form of a bolus, and provides a distinction between the vasculature and other anatomical features by virtue of its attenuation of the X-ray radiation.

Until recently, it has been assumed that the injected contrast agent has a homogeneous distribution, and consequently X-ray attenuation, in the vicinity of the interface between the contrast agent and the blood that it replaces in the vasculature. However, it has recently been found that this assumption is not always valid, and that at this interface, turbulent flow can give rise to spatial variations in the distribution of the contrast agent that are similar to those encountered when acrylic paint, or dye, is injected into water. The turbulent flow, in combination with the differing X-ray attenuation values of the contrast agent and blood, results in X-ray-based images, e.g. computed tomography "CT" images, that have a wispy and smoke-like appearance with indistinct edges and intermediate to high X-ray attenuation values. These are referred-to as "contrast agent flow artifacts". The appearance of contrast agent flow artifacts differs from the appearance of a true pathologic condition, and which is more discrete with distinct margins and lower X-ray attenuation values. Similar spatial variations in the distribution of the contrast agent, and consequently similar contrast agent flow artifacts, also occur elsewhere in the vasculature, such as at the interface between contrast agent and tissue in locations in which there is an abrupt change in fluid speed, or direction; e.g. at vessel bifurcations, stenoses, and so forth. These contrast agent flow artifacts, and also their origins, are described in a document by Robb, C. L. et al., "Evaluation and Utilization of Flow Artifacts at CT" RadioGraphics 2024; 44(5):e230134.

However, the appearance of contrast agent flow artifacts in X-ray based images can be misleading. Such artifacts are at least a distraction to a reviewing radiologist. Even worse, they risk being mis-interpreted as a true pathologic condition. Consequently, there is a need to determine the presence of contrast agent flow artifacts in medical images that are acquired using X-ray radiation.

### SUMMARY

According to one aspect of the present disclosure, a system for determining a presence of contrast agent flow artifacts, is provided. The system includes one or more processors configured to:
receive X-ray attenuation data, the X-ray attenuation data representing, at each of a plurality of different points in time, a spatial distribution of contrast agent within a vascular region;
analyze the X-ray attenuation data to identify one or more differences between the spatial distributions; and
determine a presence of at least one contrast agent flow artifact in the vascular region based on the one or more differences.

The above-described system uses differences between temporally-separated spatial distributions of contrast agent to determine the presence of contrast agent flow artifacts. The use of such differences to determine the presence of contrast agent flow artifacts is based on the insight that contrast agent flow artifacts are typically caused by turbulent flow, which by virtue of its time-dependence, gives rise to differences between the temporally-separated spatial distributions of the contrast agent. By contrast, contrast agent flow arising from a true pathology does not give rise to such differences because a true pathology exists all the time. The system may therefore be used to distinguish a contrast agent flow artifact from a true pathologic condition. In other words, it helps to reduce the risk that a contrast agent flow artifact is mis-interpreted as a true pathologic condition.

Further aspects, features, and advantages of the present disclosure will become apparent from the following description of examples, which is made with reference to the accompanying drawings.

### BRIEF DESCRIPTION OF THE DRAWINGS

Fig. 1 is a schematic diagram illustrating an example of a system 100 for determining a presence of contrast agent flow artifacts, in accordance with some aspects of the present disclosure.
Fig. 2 is a flowchart illustrating an example of a computer-implemented method of determining a presence of contrast agent flow artifacts, in accordance with some aspects of the present disclosure.
Fig. 3 is a schematic diagram illustrating an example of a vascular region including contrast agent flow artifacts 150, in accordance with some aspects of the present disclosure.
Fig. 4 is a schematic diagram illustrating an example of a reconstructed image slice representing a spatial distribution 130₁ of contrast agent at a first point in time, in accordance with some aspects of the present disclosure.
Fig. 5 is a schematic diagram illustrating an example of a reconstructed image slice representing a spatial distribution 130₂ of contrast agent at a second point in time, in accordance with some aspects of the present disclosure.
Fig. 6 is a schematic diagram illustrating an example of a difference image 160 including an identification of a contrast agent flow artifact 150, in accordance with some aspects of the present disclosure.

### DETAILED DESCRIPTION

Examples of the present disclosure are provided with reference to the following description and Figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a system, may be implemented in a computer-implemented method, and in a computer program product, in a corresponding manner.

In the following description, reference is made to examples of a system for determining the presence of contrast agent flow artifacts. In some examples, reference is made to determining the presence of contrast agent flow artifacts within the cardiac vasculature. It is, however, to be appreciated that the system may be used to determine the presence of contrast agent flow artifacts within the vasculature in general. Thus, the system may be used to determine the presence of contrast agent flow artifacts that are within other regions of the vasculature, such as within the lung, or the brain, or the arm, or the leg, and so forth.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid-state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

It is also noted that some operations that are described as being performed by the one or more processors of the systems disclosed herein may be implemented using artificial intelligence techniques. Suitable techniques may include machine learning techniques, deep learning techniques, and neural networks. For instance, one or more neural networks, may be trained in a supervised, or in some cases unsupervised, manner, to implement the operations that are performed by the one or more processors.

As mentioned above, there is a need to determine the presence of contrast agent flow artifacts in medical images that are acquired using X-ray radiation.

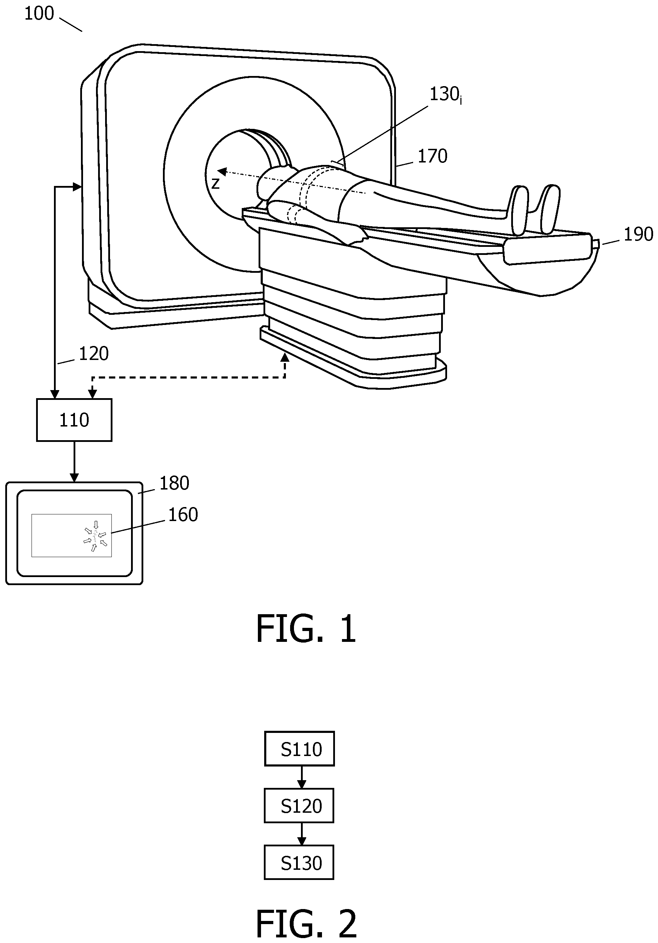

Fig. 1 is a schematic diagram illustrating an example of a system 100 for determining a presence of contrast agent flow artifacts, in accordance with some aspects of the present disclosure. Fig. 2 is a flowchart illustrating an example of a computer-implemented method of determining a presence of contrast agent flow artifacts, in accordance with some aspects of the present disclosure. It is noted that operations that are described as being performed by the one or more processors 110 of the system 100 illustrated in Fig. 1, may also be performed in the method illustrated in Fig. 2. Likewise, operations that are described in relation to the method described with reference to Fig. 2 may also be performed by the one or more processors 110 of the system 100 illustrated in Fig. 1. With reference to Fig. 1, and Fig. 2, the system 100 for determining a presence of contrast agent flow artifacts comprises one or more processors 110 configured to:
receive S110 X-ray attenuation data 120, the X-ray attenuation data representing, at each of a plurality of different points in time, i, a spatial distribution 130ᵢ of contrast agent within a vascular region;
analyze S120 the X-ray attenuation data 120 to identify one or more differences 140ⱼ between the spatial distributions 130ᵢ; and
determine S130 a presence of at least one contrast agent flow artifact 150 in the vascular region based on the one or more differences 140ⱼ.

The above-described system uses differences between temporally-separated spatial distributions of contrast agent to determine the presence of contrast agent flow artifacts. The use of such differences to determine the presence of contrast agent flow artifacts is based on the insight that contrast agent flow artifacts are typically caused by turbulent flow, which by virtue of its time-dependence, gives rise to differences between the temporally-separated spatial distributions of the contrast agent. By contrast, contrast agent flow arising from a true pathology does not give rise to such differences because a true pathology exists all the time. The system may therefore be used to distinguish a contrast agent flow artifact from a true pathologic condition. In other words, it helps to reduce the risk that a contrast agent flow artifact is mis-interpreted as a true pathologic condition.

The operations that are performed by the processor(s) of the system 100 are described in more detail below.

Referring initially to the operation S110; in this operation, X-ray attenuation data 120 is received. The X-ray attenuation data represents, at each of a plurality of different points in time, i, a spatial distribution 130ᵢ of contrast agent within a vascular region.

The X-ray attenuation data 120 that is received in the operation S110 may be acquired using various types of X-ray based imaging systems, some of which are described below. In general, the X-ray attenuation data 120 that is received in the operation S120 may be raw data, i.e. projection data, or it may be image data; i.e. projection data that has been reconstructed into an image.

In some examples, the X-ray attenuation data 120 comprises computed tomography, CT, X-ray attenuation data, i.e. X-ray attenuation data representing a volumetric, or 3D, image. In other examples, the X-ray attenuation data 120 comprises spectral CT X-ray attenuation data, i.e. X-ray attenuation data representing a volumetric, or 3D, image and wherein the data represents X-ray attenuation within each of a plurality of different energy intervals DE_{1..m}.

In general, (spectral) CT X-ray attenuation data may be generated by a (spectral) CT imaging system. Alternatively, as described in more below, (spectral) CT X-ray attenuation data may be generated by rotating, or stepping the X-ray source and X-ray detector of a (spectral) X-ray projection imaging system around the vascular region.

A CT imaging system generates CT X-ray attenuation data by rotating, or stepping, an X-ray source and an X-ray detector around a vascular region and acquiring X-ray attenuation data for the vascular region from multiple rotational angles with respect to the vascular region. The CT X-ray attenuation data may then be reconstructed into a 3D image of the vascular region. Examples of CT imaging systems that may be used to generate CT X-ray attenuation data include cone beam CT imaging systems, photon counting CT imaging systems, dark-field CT imaging systems, and phase contrast CT imaging systems.

An example of a CT imaging system 170 that may be used to generate CT X-ray attenuation data, is illustrated in Fig. 1. In general, the CT imaging system 170 may be a multi-slice CT imaging system, or a single-slice CT imaging system. The CT imaging system may be operated in various imaging modes, such as in an axial imaging mode, or in a stepped axial "i.e. step-and-shoot" imaging mode, or in a helical imaging mode. By way of an example, CT X-ray attenuation data that is received in the operation S110 may be generated by the CT 5000 Ingenuity CT imaging system that is marketed by Philips Healthcare, Best, The Netherlands.

As mentioned above, CT X-ray attenuation data may alternatively be generated by rotating, or stepping the X-ray source and X-ray detector of an X-ray projection imaging system around a vascular region. An X-ray projection imaging system may include a support arm such as a so-called "C-arm" that supports its X-ray source and X-ray detector. In contrast to a CT imaging system, an X-ray projection imaging system typically generates X-ray attenuation data for a vascular region with its X-ray source and X-ray detector in a static position with respect to the vascular region. The X-ray attenuation data may be referred-to as projection data. The X-ray attenuation data that is generated by an X-ray projection imaging system is typically used to generate a 2D image of the vascular region. However, an X-ray projection imaging system may generate CT X-ray attenuation data, i.e. volumetric data, by rotating, or stepping, its X-ray source and X-ray detector around a vascular region and acquiring projection data for the vascular region from multiple rotational angles with respect to the vascular region. Image reconstruction techniques may then be used to reconstruct the projection data that is obtained from the multiple rotational angles into a volumetric image in a similar manner to the reconstruction of a volumetric image using X-ray attenuation data that is acquired from a CT imaging system. Thus, CT X-ray attenuation data that is received in the operation S110, may be generated by a CT imaging system, or alternatively, it may be generated by an X-ray projection imaging system. An example of an X-ray projection imaging system that may be used to generate CT X-ray attenuation data, is the Azurion 7 X-ray projection imaging system that is marketed by Philips Healthcare, Best, The Netherlands.

As mentioned above, in some examples, the X-ray attenuation data 120 that is received in the operation S110 includes spectral CT X-ray attenuation data. Spectral CT X-ray attenuation data defines X-ray attenuation within each of a plurality of different energy intervals DE_{1..m}. In general there may be two or more energy intervals; i.e. *m* is an integer, and *m* ≥ 2. Spectral CT X-ray attenuation data may be generated by a spectral CT imaging system, or by a spectral X-ray projection imaging system. In the latter case, the spectral CT X-ray attenuation data may be acquired by rotating, or stepping the X-ray source and X-ray detector of the spectral X-ray projection imaging system around the vascular region, as described above. More generally, spectral CT X-ray attenuation data may be generated by a spectral X-ray imaging system.

The ability to generate X-ray attenuation data at multiple different energy intervals distinguishes a spectral X-ray imaging system from a conventional X-ray imaging system. By processing the data from the multiple different energy intervals, a distinction can be made between media that have similar X-ray attenuation values when measured within a single energy interval, and which would be indistinguishable in conventional X-ray attenuation data. Examples of spectral X-ray imaging systems that may be used to generate spectral CT X-ray attenuation data include cone beam spectral X-ray imaging systems, photon counting spectral X-ray imaging systems, dark-field spectral X-ray imaging systems, and phase contrast spectral X-ray imaging systems. An example of a spectral CT imaging system that may be used to generate spectral CT X-ray attenuation data is the Spectral CT 7500 that is marketed by Philips Healthcare, Best, The Netherlands.

Spectral CT X-ray attenuation data may be generated by various different configurations of spectral X-ray imaging systems. For instance, the X-ray source of a spectral X-ray imaging system may include multiple monochromatic sources, or one or more polychromatic sources, and the X-ray detector of a spectral X-ray imaging system may include: a common detector for detecting multiple different X-ray energy intervals, or multiple detectors wherein each detector detects a different X-ray energy interval DE_{1..m}, or a multi-layer detector in which X-rays having energies within different X-ray energy intervals are detected by corresponding layers, or a photon counting detector that bins detected X-ray photons into one of multiple energy intervals based on their individual energies.

The X-ray attenuation data 120 that is received in the operation S110 represents, at each of a plurality of different points in time, i, a spatial distribution 130ᵢ of contrast agent within a vascular region.

The X-ray attenuation data 120 that is received in the operation S110 is therefore generated subsequent to the injection of a contrast agent into the vasculature of a subject. The contrast agent may include a substance such as Iodine, or a Lanthanide such as Gadolinium, or another substance that provides visibility of a blood flow into which the contrast agent is injected. The contrast agent may be injected manually or automatically, e.g. via an injector. The contrast agent may be injected in the form of a bolus, for example.

In general, the X-ray attenuation data 120 that is received in the operation S110 represents, at each of two or more points in time, i, a spatial distribution 130ᵢ of contrast agent within corresponding portion(s) of a vascular region. The portion(s) may be volume(s), or image slice(s), for example. By way of an example, the X-ray attenuation data 120 may represent, at each of two or more points in time, i, a spatial distribution 130ᵢ of contrast agent within an image slice through the heart, as illustrated in Fig. 1. Such X-ray attenuation data may be generated by a single-slice (spectral) CT imaging system, for example. By way of another example, the X-ray attenuation data 120 may represent, at each of two or more points in time, i, a spatial distribution 130ᵢ of contrast agent within multiple image slices through the heart. Such X-ray attenuation data may be generated by a multi-slice (spectral) CT imaging system, such as a 64-slice CT imaging system for example.

In the analyzing operation S120 that is described in more detail below, differences between spatial distributions at the different points in time, i, and for corresponding portion(s) of the vascular region, are then identified and used to determine the presence of contrast agent flow artifacts. Thus, spatial distributions at two or more points in time, i, and for corresponding portion(s) of the vascular region (e.g. image slice(s)), are required in order to perform the analysis in the operation S120. In some examples, the X-ray attenuation data 120 that is received in the operation S110 represents, at each of three or more points in time, i, a spatial distribution 130ᵢ of contrast agent within corresponding portion(s) of a vascular region. If spatial distributions at three or more points in time, i, and for corresponding portion(s) of the vascular region are used to perform the analysis in the operation S120, the presence of a contrast agent flow artifact that is determined on the basis of differences between two spatial distributions for corresponding portion(s) of the vascular region, may be confirmed via additional differences that occur between the larger set of spatial distributions.

In general, the X-ray attenuation data 120 for the spatial distributions 130ᵢ is acquired over a time interval during which it is expected that there will be negligible changes in the spatial distributions. In some imaging protocols, contrast agent is injected into a subject and allowed to reach a homogeneous distribution in a vascular region, e.g. the aorta, before imaging commences. Negligible changes in the contrast agent distribution are then expected for the duration of the (cardiac) imaging procedure. The plurality of points in time, i, may therefore occur during a same imaging procedure. The plurality of points in time, i, may occur within a shorter time period, such as a few seconds. By way of a non-limiting example, a time interval separating consecutive points in time may be in the range from approximately 10 milliseconds to approximately 1 second.

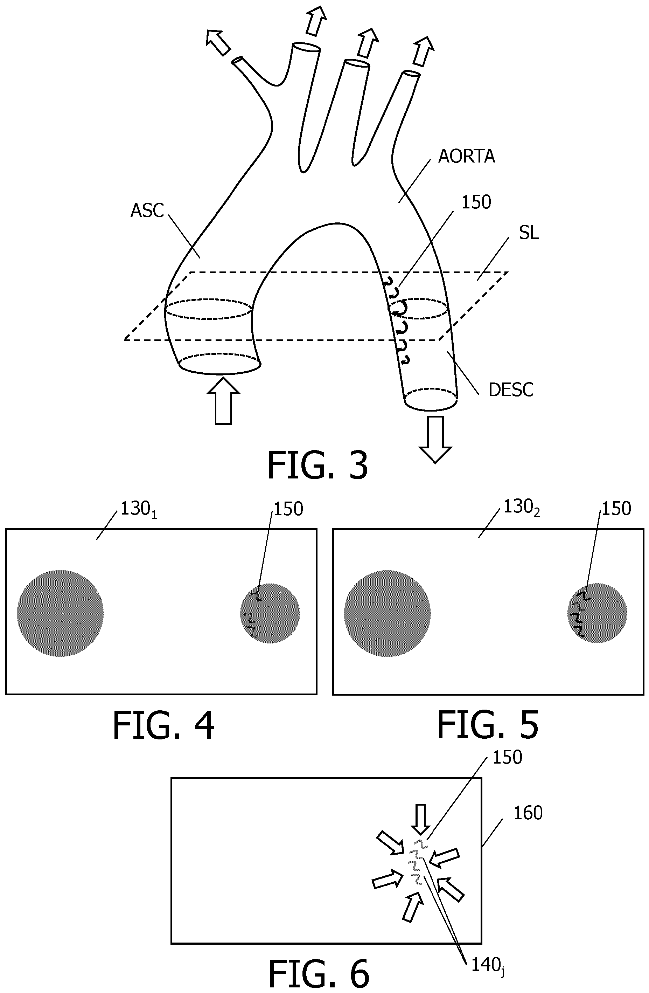

Some examples of spatial distributions of contrast agent within a vascular region are now described with reference to Fig. 3 - Fig. 5. Fig. 3 is a schematic diagram illustrating an example of a vascular region including contrast agent flow artifacts 150, in accordance with some aspects of the present disclosure. The vascular region represented in Fig. 3 includes a portion of the aorta, AORTA. The blood flow direction within the aorta is illustrated via arrows. Thus, blood flows into the ascending aorta, ASC, and out of the descending aorta, DESC. Contrast agent (not illustrated) has been injected into the vasculature in order to provide visibility of the flow. As illustrated in Fig. 3, the path of the aorta is steeply curved between the ascending and descending portions, ASC and DESC. As described above, this abrupt change in direction gives rise to an abrupt change in fluid speed at the interface between the contrast agent and the inner surface of the descending aorta. This, in turn, results in turbulent flow at this interface, as illustrated by the dark circular-shaped arrows in Fig. 3. The turbulent flow, in combination with the differing X-ray attenuation values of the contrast agent and blood, results in temporal variations in the spatial distribution of contrast agent at this interface. Such temporal variations may be observed in the spatial distribution of contrast agent across the image slice SL, for example.

Some examples illustrating temporal variations in the spatial distribution of contrast agent across the image slice SL are described with reference to Fig. 4, and Fig. 5. Fig. 4 is a schematic diagram illustrating an example of a reconstructed image slice representing a spatial distribution 130₁ of contrast agent at a first point in time, in accordance with some aspects of the present disclosure. Fig. 5 is a schematic diagram illustrating an example of a reconstructed image slice representing a spatial distribution 130₂ of contrast agent at a second point in time, in accordance with some aspects of the present disclosure. In this example, the second point in time is later than the first point in time. The second point in time may be a few hundred milliseconds after the first point in time, for example. The reconstructed images illustrated in Fig. 4 and Fig. 5 may be generated by reconstructing X-ray attenuation data corresponding to the image slice SL using known image reconstruction techniques. In Fig. 4 and Fig. 5, contrast agent is illustrated with dark shading. Within the ascending aorta illustrated on the left-hand side of Fig. 4 and Fig. 5, contrast agent is present and there are no contrast agent flow artifacts. Consequently, the ascending aorta illustrated in the left-hand dark-shaded regions in Fig. 4 and Fig. 5 is illustrated with uniform shading, and which represents a homogeneous contrast agent distribution. Within the descending aorta illustrated on the right-hand side of Fig. 4 and Fig. 5, however, the turbulent flow described above results in contrast agent flow artifacts that have a wispy and smoke-like appearance. These contrast agent flow artifacts are illustrated as darker lines within an otherwise homogeneous contrast agent distribution in the right-hand dark-shaded regions in Fig. 4 and Fig. 5. Due to the time-varying nature of turbulence, the contrast agent flow artifacts in Fig. 4 appear different to those in Fig. 5. For instance, the magnitude, and also the number, of contrast agent flow artifacts increases from Fig. 4 to Fig. 5.

The X-ray attenuation data 120 representing the spatial distributions 130ᵢ illustrated in Fig. 4 and Fig. 5 may be acquired using the imaging systems described above. Various imaging modes may be used with such imaging systems to acquire the X-ray attenuation data. For instance, in one example, the X-ray attenuation data 120 is generated by a single/ multi-slice (spectral) CT imaging system that is operated in an axial imaging mode. A single/ multi-slice (spectral) CT imaging system may be used to acquire X-ray attenuation data representing one or more spatial distributions 130ᵢ of contrast agent within a vascular region, i.e. "image slice(s)", in each rotation of their X-ray source and X-ray detector around the vascular region. If the axial position of the vascular region in relation to the X-ray source and X-ray detector is unchanged during imaging, then X-ray attenuation data for the same image slice(s), i.e. corresponding portion(s) of the vascular region, is acquired by the same area of the X-ray detector in consecutive rotations. Thus, if the X-ray attenuation data 120 is acquired in the axial imaging mode, a time interval between the acquisition of data for consecutive spatial distributions is determined by the revolution period of the X-ray source and X-ray detector.

A single/ multi-slice (spectral) CT imaging system may also be operated in a stepped axial imaging mode wherein the axial position of the vascular region in relation to the X-ray source and X-ray detector, or vice versa, is adjusted in steps during imaging. **In** the stepped axial imaging mode, a multi-slice (spectral) CT imaging system may be used to acquire X-ray attenuation data for the same image slice(s), i.e. corresponding portion(s) of the vascular region, in consecutive rotations using different areas of the X-ray detector. If the X-ray attenuation data 120 is acquired in the stepped axial imaging mode, a time interval between the acquisition of data for consecutive spatial distributions is determined by the time interval between consecutive steps.

A single/ multi-slice (spectral) CT imaging system may also be operated in a helical imaging mode wherein the axial position of the vascular region in relation to the X-ray source and X-ray detector, or vice versa, is adjusted continuously during imaging. **In** the helical imaging mode, a multi-slice (spectral) CT imaging system may be used to acquire X-ray attenuation data for the same image slice(s), i.e. corresponding portion(s) of the vascular region, in consecutive revolutions using different areas of the X-ray detector. If the X-ray attenuation data 120 is acquired in the helical imaging mode, a time interval between the acquisition of data for consecutive spatial distributions is determined by the revolution period of the X-ray source and X-ray detector.

By way of a non-limiting example, the X-ray attenuation data 120 may be acquired by a 64-slice (spectral) CT imaging system with a revolution period in the order of a few hundred milliseconds. The CT imaging system may be operated in a helical imaging mode with a pitch factor of approximately 0.5. **In** this imaging mode, the X-ray attenuation data corresponding to the different points in time may be acquired during different revolutions of the X-ray source and X-ray detector around the vascular region, and using different areas of the X-ray detector. Aperture weighting may be used in the subsequent reconstruction of the image slices in order to reduce image artifacts arising from X-ray beam divergence.

An advantage of acquiring the X-ray attenuation data 120 using the above-described axial, stepped axial, and likewise helical, imaging modes, is that these are standard imaging modes. Thus, the X-ray attenuation data that is used by the system described herein does not require any change to the conventional method of acquiring X-ray attenuation data. Thus, it may be adopted seamlessly into an existing imaging workflow.

In general, the X-ray attenuation data 120 that is received in the operation S110 may be received via any form of data communication, including wired, optical, and wireless communication. By way of some examples, when wired or optical communication is used, the communication may take place via signals transmitted on an electrical or optical cable, and when wireless communication is used, the communication may for example be via RF or optical signals. The X-ray attenuation data 120 that is received in the operation S110 may be received from various sources. For example, the X-ray attenuation data 120 may be received from an imaging system, such as one of the imaging systems described above. Alternatively, the X-ray attenuation data 120 may be received from another source, such as a computer readable storage medium, the Internet, or the Cloud, for example.

Returning to the method described with reference to Fig. 2; in the operation S120, the X-ray attenuation data 120 is analyzed to identify one or more differences 140ⱼ between the spatial distributions 130ᵢ.

In one example, the one or more differences 140ⱼ that are identified in the operation S120 include changes over time in X-ray attenuation values at corresponding locations within the vascular region. The differences may be evaluated by comparing the intensities at one or more corresponding point(s) within the vascular region. Alternatively, the differences may be evaluated by comparing the intensities within one or more corresponding sub-regions. In the latter case, the difference(s) may be evaluated per sub-region, e.g. as an average of the X-ray attenuation values in the sub-region. For instance, they may be evaluated as an average of the X-ray intensity values for a portion of the aorta.

In one example, the one or more differences 140ⱼ occur at an interface between the contrast agent and blood or tissue within the vascular region. As described above, such interfaces are susceptible to contrast agent flow artifacts as a result of turbulence. The tissue may form part of a vessel wall, for example. If the contrast agent is injected in the form of a bolus, the one or more differences 140ⱼ may occur behind a front of the bolus, for example.

In one example, the one or more differences 140ⱼ occur within a vascular region that has been filled with contrast agent. The vascular region may be a portion of the aorta, for example.

The differences 140ⱼ may be identified in various ways in the operation S120. In one example, the operation of analyzing S120 the X-ray attenuation data 120 to identify one or more differences 140ⱼ between the spatial distributions 130ᵢ, comprises:
reconstructing, for each of the points in time, i, an image representing the spatial distribution 130ᵢ; and
generating a difference image 160 identifying the one or more differences 140ⱼ by subtracting one of the reconstructed images from another of the reconstructed images.

This example is described with reference to Fig. 4 - Fig. 6. As described above, the images illustrated in Fig. 4 and Fig. 5 show examples of reconstructed image slices that represent spatial distributions, 130₁, 130₂ of contrast agent at different points in time. The reconstructed image slices represent the spatial distributions through the same portion of a vascular region, i.e. the slice SL illustrated in Fig. 3. In this example, a difference image 160 is generated by subtracting one of the reconstructed images from another of the reconstructed images. Fig. 6 is a schematic diagram illustrating an example of a difference image 160 including an identification of a contrast agent flow artifact 150, in accordance with some aspects of the present disclosure. The image illustrated in Fig. 6 is generated by subtracting the image illustrated in Fig. 4 from the image illustrated in Fig. 5. The intensity value across most of the area of the difference image is negligible because there is negligible change in the distribution of the contrast agent in the time interval between the two images. Hence, the image appears white within the ascending aorta ASC and also across much of the descending aorta DESC. However, as described above, there are non-negligible differences 140ⱼ in the spatial distributions at the interface between contrast agent and the inner surface of the descending aorta DESC. These non-negligible differences 140ⱼ indicate the presence of contrast agent flow artifacts. These differences 140ⱼ are illustrated as grey lines in the difference image 160 shown in Fig. 6.

One or more further operations may be performed in order to improve the reliability of the difference image 160 described above. For instance, in one example, the one or more processors 110 perform a de-noising operation on the reconstructed images and/or on the difference image 160. Known de-noising techniques may be used to perform this operation. The de-noising operation(s) improve the image quality of the difference image, and consequently its reliability.

In another example, the one or more processors mutually register the reconstructed images prior to generating the difference image 160. Known rigid and/or non-rigid image registration techniques may be used to register the images. This registration improves the reliability of the images by ensuring that corresponding regions of the images are aligned prior to generating the difference image 160.

In a related example, if the X-ray attenuation data 120 includes spectral CT X-ray attenuation data, the image registration in this example may be performed using virtual images from which contrast agent has been excluded. Using the virtual images to perform this registration reduces that risk that contrast agent flow artifacts in the images themselves distort the registration and thereby gives a misleading difference image.

As mentioned above, the difference(s) 140ⱼ between the spatial distributions 130ᵢ may be identified in other ways than generating a difference image in the operation S120. For instance, various machine learning techniques may be used to determine the difference(s). By way of an example, a neural network may be trained to identify the difference(s) 140ⱼ by training the neural network with training data that includes multiple pairs of spatial distributions of contrast agent with varying degrees of differences, together with ground truth data indicating the differences.

Returning now to the operation S130, illustrated in Fig. 2, in this operation, the presence of at least one contrast agent flow artifact 150 in the vascular region is determined based on the one or more differences 140ⱼ.

The operation S130 may be performed in various ways. For instance, in one example, the operation of determining S130 a presence of at least one contrast agent flow artifact 150 in the vascular region comprises outputting an indication of a presence and/ or a magnitude of the at least one contrast agent flow artifact. This may be performed by outputting the difference image 160 illustrated in Fig. 6, for example. The difference image 160 inherently indicates the location of the contrast agent flow artifact(s). The difference image 160 also indicates the magnitude of the contrast agent flow artifact(s) via the image intensity. The difference image 160 may appear substantially blank, or substantially with image intensities that correspond to noise image, and wherein significant deviations from the noise that correspond to contrast agent flow artifacts are shown with a significantly different intensity. Noise reduction algorithms might be applied to further increase the Contrast-to-Noise Ratio "CNR" of the flow artifacts in the difference images. Any appropriate mapping, e.g. an intensity mapping, or a color mapping, may be used to display the values in the difference image 160. If the difference image 160 includes negative values as a result of reductions in the magnitude of the image intensities over time at the locations of the contrast agent flow artifacts, such negative values may be displayed. Alternatively, an image intensity mapping, or color mapping, may be used wherein the magnitude of the difference(s) is displayed.

In a related example, the location of the above-described significant intensity deviations may be highlighted in order to draw a viewer's attention to the location of the contrast agent flow artifacts. The highlighting may be performed in various ways. For instance, a boundary may be provided in the difference image that indicates the location of the contrast agent flow artifacts, or, alternatively, arrows that point to the contrast agent flow artifacts 150 may be included in the difference image 160, as shown in Fig. 6.

In another example, the operation of determining S130 a presence of at least one contrast agent flow artifact 150 in the vascular region comprises:
comparing the one or more differences 140ⱼ with a threshold value; and
confirming the presence of the at least one contrast agent flow artifact 150 based on the one or more differences 140ⱼ exceeding the threshold value.

The use of the threshold value in this example facilitates a clearer distinction between contrast agent flow artifacts and other image artifacts. The threshold value may be set to a level that enables contrast agent flow artifacts to be distinguished from image noise, for example. **In** a related example, an alert may be generated if the threshold value is exceeded. For example, a warning symbol, or message, may be outputted to a display device, or an audible alert, may be outputted to an audio device.

One or more further operations may be performed in the method illustrated in Fig. 2. In one example, the one or more processors 110 are further configured to:
output an image representation of the vascular region; and
identify a location of the at least one contrast agent flow artifact 150 in the outputted image representation.

The outputted image representation enables a user to readily determine the location of the contrast agent flow artifacts. This reduces the risk that contrast agent flow artifacts are mis-interpreted as a true pathologic condition.

The image representation may be outputted in various ways, including to a display device such as a monitor, or to a virtual/ augmented reality display device; or to a printer; or to a computer-readable storage medium; or to the Internet; or to the Cloud, and so forth. In general, the image representation may be provided in the form of a single image, or it may be provided in the form of a video showing the differences over time. By way of an example, the image representation may be outputted to the monitor 180 illustrated in Fig. 1.

In this example, the image representation may for instance include:
a reconstructed image representing the spatial distribution 130ᵢ of contrast agent within the vascular region at one of the points in time;
   and/or
a difference image 160 representing the one or more differences 140ⱼ over a time interval separating the points in time, i.

In this example, the reconstructed image, and likewise the difference image 160, may be generated using the techniques described above.

In a related example, image representation comprises the reconstructed image and the difference image 160, and the difference image 160 is overlaid on the reconstructed image.

This image representation provides an intuitive visualization of the contrast agent flow artifacts.

In another example, the one or more processors 110 are configured to output the image representation of the vascular region selectively for only a portion of the vascular region.

The image representation that is provided by this example facilitates a user to focus on the selected portion of the vascular region without being distracted by other image features, such as anatomical features, motion-induced changes in the images, contrast agent flow artifacts in other anatomical regions, and so forth. In this example, the image representation may be outputted for only a portion of the aorta, or for only a portion of a specific vessel, for example.

In another example, the one or more processors 110 are further configured to one or more of:
localize and/or characterize a vascular defect in the vascular region based on the one or more differences 140ⱼ;
   and
determine a blood flow direction and/or a blood flow velocity within the vascular region based on the one or more differences 140ⱼ; and
output in the image representation an indication of the blood flow direction.

In this example, a vascular defect may be identified based on the continuous appearance of an image feature in the spatial distributions 130ᵢ. As described above, a true pathology exists all the time and so its appearance does not vary significantly between the spatial distributions 130ᵢ. In this example, a blood flow direction may be determined based on the one or more differences 140ⱼ by analyzing the direction in which the differences move over time. In this example, three or more spatial distributions may be used to provide a more accurate assessment of the blood flow direction. In this example, a blood flow velocity may be determined by assessing the temporal characteristics of the differences, e.g. the speed of movement of the differences, or the persistence time of the differences.

It is noted that in addition to the one or more processors 110, the system 100 may also include one or more of: an X-ray based imaging system for providing the X-ray attenuation data 120, such as for example the CT imaging system 170 illustrated in Fig. 1; an injector (not shown in Fig. 1) for injecting a contrast agent into a subject; a display device such as the monitor 180 illustrated in Fig. 1, for displaying outputs generated by the one or more processors 110, such as an indication of a presence and/ or a magnitude of the contrast agent flow artifact(s), image representations of the vascular region, and so forth; a patient bed 190 for supporting a subject and thus the vascular region during acquisition of the X-ray attenuation data 120; and a user input device (not illustrated in Fig. 1) configured to receive user input in relation to the operations performed by the one or more processors 110, such as a keyboard, a mouse, a touchscreen, and so forth.

In another example, a computer-implemented method of determining a presence of contrast agent flow artifacts, is provided. The method comprises:
receiving S 110 X-ray attenuation data 120, the X-ray attenuation data representing, at each of a plurality of different points in time, i, a spatial distribution 130ᵢ of contrast agent within a vascular region;
analyzing S120 the X-ray attenuation data 120 to identify one or more differences 140ⱼ between the spatial distributions 130ᵢ; and
determining S 130 a presence of at least one contrast agent flow artifact 150 in the vascular region based on the one or more differences 140ⱼ.

The operations that are performed in the method are shown in the flowchart illustrated in Fig. 2.

**In** another example, a computer program product is provided. The computer program product comprises instructions which when executed by one or more processors 110, cause the one or more processors to carry out a method of determining a presence of contrast agent flow artifacts. The method comprises:
receiving S 110 X-ray attenuation data 120, the X-ray attenuation data representing, at each of a plurality of different points in time, i, a spatial distribution 130ᵢ of contrast agent within a vascular region;
analyzing S120 the X-ray attenuation data 120 to identify one or more differences 140ⱼ between the spatial distributions 130ᵢ; and
determining S 130 a presence of at least one contrast agent flow artifact 150 in the vascular region based on the one or more differences 140ⱼ.

The above examples are to be understood as illustrative of the present disclosure, and not restrictive. Further examples are also contemplated. For instance, the examples described in relation to a system, may also be provided by the computer-implemented method, or by the computer program product, or by the computer-readable storage medium, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A system (100) for determining a presence of contrast agent flow artifacts, the system comprising one or more processors (110) configured to:
receive (S110) X-ray attenuation data (120), the X-ray attenuation data representing, at each of a plurality of different points in time (i), a spatial distribution (130ᵢ) of contrast agent within a vascular region;
analyze (S120) the X-ray attenuation data (120) to identify one or more differences (140ⱼ) between the spatial distributions (130ᵢ); and
determine (S130) a presence of at least one contrast agent flow artifact (150) in the vascular region based on the one or more differences (140ⱼ).

2. The system according to claim 1, wherein the X-ray attenuation data (120) comprises computed tomography, CT, X-ray attenuation data, optionally spectral CT X-ray attenuation data;
and/or
wherein the X-ray attenuation data (120) is generated by a multi-slice CT imaging system and wherein the spatial distributions (130ᵢ) of contrast agent represent image slices through corresponding portions of the vascular region.

3. The system according to claim 1 or claim 2, wherein the X-ray attenuation data (120) is generated by an X-ray source and X-ray detector during a helical CT imaging mode; and
wherein the X-ray attenuation data (120) corresponding to the different points in time (i) is acquired during different revolutions of the X-ray source and X-ray detector around the vascular region and and/or using different areas of the X-ray detector.

4. The system according to any previous claim, wherein the analyzing (S120) the X-ray attenuation data (120) comprises:
reconstructing, for each of the points in time (i), an image representing the spatial distribution (130ᵢ); and
generating a difference image (160) identifying the one or more differences (140ⱼ) by subtracting one of the reconstructed images from another of the reconstructed images.

5. The system according to any previous claim, wherein the one or more differences (140ⱼ) comprise changes over time in X-ray attenuation values at corresponding locations within the vascular region.

6. The system according to any previous claim, wherein the one or more differences (140ⱼ) occur at an interface between the contrast agent and blood or tissue within the vascular region.

7. The system according to any previous claim, wherein the determining (S130) a presence of at least one contrast agent flow artifact (150) in the vascular region comprises:
comparing the one or more differences (140ⱼ) with a threshold value; and
confirming the presence of the at least one contrast agent flow artifact (150) based on the one or more differences (140ⱼ) exceeding the threshold value.

8. The system according to any previous claim, wherein the determining (S130) a presence of at least one contrast agent flow artifact (150) in the vascular region comprises outputting an indication of a presence and/ or a magnitude of the at least one contrast agent flow artifact.

9. The system according to any previous claim, wherein the one or more processors (110) are further configured to:
output an image representation of the vascular region; and
identify a location of the at least one contrast agent flow artifact (150) in the outputted image representation.

10. The system according to claim 9, wherein the image representation comprises:
a reconstructed image representing the spatial distribution (130ᵢ) of contrast agent within the vascular region at one of the points in time;
and/or
a difference image (160) representing the one or more differences (140ⱼ) over a time interval separating the points in time (i).

11. The system according to claim 10 wherein the image representation comprises the reconstructed image and the difference image (160); and
wherein the difference image (160) is overlaid on the reconstructed image.

12. The system according to any one of claims 9 - 11, wherein the one or more processors (110) are configured to output the image representation of the vascular region selectively for only a portion of the vascular region.

13. The system according to any one of claims 9 - 12, wherein the one or more processors (110) are further configured to one or more of:
localize and/or characterize a vascular defect in the vascular region based on the one or more differences (140ⱼ);
and
determine a blood flow direction and/or a blood flow velocity within the vascular region based on the one or more differences (140ⱼ); and
output in the image representation an indication of the blood flow direction.

14. A computer-implemented method of determining a presence of contrast agent flow artifacts, the method comprising:
receiving (S110) X-ray attenuation data (120), the X-ray attenuation data representing, at each of a plurality of different points in time (i), a spatial distribution (130ᵢ) of contrast agent within a vascular region;
analyzing (S120) the X-ray attenuation data (120) to identify one or more differences (140ⱼ) between the spatial distributions (130ᵢ); and
determining (S130) a presence of at least one contrast agent flow artifact (150) in the vascular region based on the one or more differences (140ⱼ).

15. A computer program product comprising instructions which when executed by one or more processors (110), cause the one or more processors to carry out the method according to claim 14.
